(19) Europäisches Patentamt
European Patent Office
Office européen des brevets

(11) Numéro de publication : **0 034 989**
**B1**

(12) # FASCICULE DE BREVET EUROPÉEN

(45) Date de publication du fascicule du brevet : **05.10.83**

(51) Int. Cl.³ : **C 07 D209/94**, A 61 K 31/40

(21) Numéro de dépôt : **81400273.9**

(22) Date de dépôt : **20.02.81**

(54) **Nouveaux dérivés du cycloheptindolol et leurs sels avec les acides, leur préparation, leur application comme médicaments et les compositions les renfermant.**

(30) Priorité : **26.02.80 FR 8004198**
**18.11.80 FR 8024406**

(43) Date de publication de la demande :
**02.09.81 Bulletin 81/35**

(45) Mention de la délivrance du brevet :
**05.10.83 Bulletin 83/40**

(84) Etats contractants désignés :
**AT BE CH DE FR GB IT LI LU NL SE**

(56) Documents cités :
**EP A 0 002 570**
**FR A 1 179 965**

**HELVETICA CHIMICA ACTA, 1952, vol. 35, pages 148-152 Basel, CH**

(73) Titulaire : **ROUSSEL-UCLAF**
**35, boulevard des Invalides**
**F-75007 Paris (FR)**

(72) Inventeur : **Nedelec, Lucien**
**45, boulevard de l'Ouest**
**F-93340 Le Raincy (FR)**
Inventeur : **Frechet, Daniel**
**45, rue Lecourbe**
**F-75015 Paris (FR)**
Inventeur : **Dumont, Claude**
**33, rue du Maréchal Vaillant**
**F-94130-Nogent sur Marne (FR)**
Inventeur : **Plassard, Guy**
**6, rue Clément Marot**
**F-91600 Savigny sur Orge (FR)**
Inventeur : **Brown, Neil Leslie**
**12, rue Jaucourt**
**F-75012 Paris (FR)**

(74) Mandataire : **Douetteau, Pierre et al**
**c/o ROUSSEL-UCLAF 102, route de Noisy Boite postale 9**
**F-93230 Romainville (FR)**

EP 0 034 989 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Nouveaux dérivés du cycloheptindolol et leurs sels avec les acides, leur préparation, leur application comme médicaments et les compositions les renfermant

La présente invention concerne de nouveaux dérivés du cycloheptindolol et leurs sels avec les acides, leur préparation, leur application comme médicaments et les compositions pharmaceutiques les renfermant.

L'invention a pour objet les nouveaux dérivés du cycloheptindolol, répondant à la formule générale I :

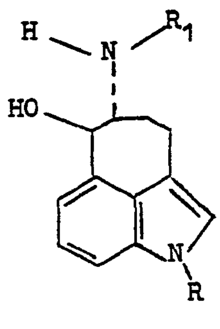

(I)

dans laquelle R représente un atome d'hydrogène, un radical alcoyle renfermant de 1 à 5 atomes de carbone ou un radical aralcoyle renfermant de 7 à 12 atomes de carbone, éventuellement substitué par des substituants chloro, bromo, méthyl, éthyl, méthoxy, trifluorométhyl ou méthylthio, et $R_1$ représente un atome d'hydrogène, un radical alcoyle renfermant de 1 à 5 atomes de carbone, cycloalcoylaloyle renfermant de 4 à 7 atomes de carbone ou aralcoyle renfermant de 7 à 12 atomes de carbone, éventuellement substitué par des substituants chloro, bromo, méthyl, éthyl, méthoxy, trifluorométhyl ou méthylthio ainsi que les sels d'addition avec les acides minéraux ou organiques desdits dérivés.

Dans la formule générale I et dans ce qui suit, le terme radical alcoyle renfermant de 1 à 5 atomes de carbone désigne, par exemple, un radical méthyl, éthyl, propyl, isopropyl, butyl, isobutyl ou pentyl ; le terme radical aralcoyle renfermant de 7 à 12 atomes de carbone éventuellement substitué désigne, par exemple, un radical benzyl ou phénéthyl, dont les substituants peuvent être des radicaux chloro, bromo, méthyl, éthyl, méthoxy, trifluorométhyl, ou méthylthio ; le terme radical cycloalcoylalcoyle renfermant de 4 à 7 atomes de carbone désigne, par exemple, un radical cyclopropylméthyl.

Les sels d'addition avec les acides minéraux ou organiques peuvent être, par exemple, les sels formés avec les acides chlorhydrique, bromhydrique, iodhydrique, nitrique, sulfurique, phosphorique, acétique, formique, propionique, benzoïque, maléique, fumarique, succinique, tartrique, citrique, oxalique, glyoxylique, aspartique, alcane sulfonique, tels que l'acide méthane sulfonique et aryl sulfonique, tels que l'acide benzène sulfonique.

La liaison en pointillés indique que les substituants OH et $NHR_1$ sont en position trans.

Parmi les produits, objet de l'invention, on peut citer, notamment, les dérivés du cycloheptindolol répondant à la formule I ci-dessus dans laquelle R et $R_1$ représentent un atome d'hydrogène ou un radical alcoyle renfermant de 1 à 5 atomes de carbone, ainsi que leurs sels d'addition avec les acides minéraux ou organiques.

Parmi les produits, objet de l'invention, on retient plus particulièrement

— le 5-R,S-trans amino 3,4,5,6-tétrahydro 1H-cyclohept [c,d] indol-6-ol ;

— le 5-R,S-trans 5-méthylamino 3,4,5,6-tétrahydro 1H-cyclohept [c,d] indol-6-ol ;

ainsi que leurs sels d'addition avec les acides minéraux ou organiques.

Les dérivés du cycloheptindolol de formule I et leurs sels possèdent de très intéressantes propriétés pharmacologiques ; ils sont doués, notamment, de remarquables propriétés hypotensives et antihypertensives.

Ces propriétés sont illustrées plus loin dans la partie expérimentale et justifient l'utilisation des dérivés de formule I et de leurs sels d'addition avec les acides pharmaceutiquement acceptables, à titre de médicaments.

La présente demande a ainsi également pour objet l'application, à titre de médicaments, des dérivés de cycloheptindolol tels que définis par la formule I, ainsi que de leurs sels d'addition avec les acides pharmaceutiquement acceptables.

Parmi les médicaments, selon l'invention, on peut citer, notamment, les dérivés du cycloheptindolol répondant à la formule I ci-dessus dans laquelle R et $R_1$ représentent un atome d'hydrogène ou un radical alcoyle renfermant de 1 à 5 atomes de carbone ainsi que leurs sels d'addition avec les acides pharmaceutiquement acceptables.

Parmi ceux-ci, on retient plus particulièrement :

— le 5-R,S-trans amino 3,4,5,6-tétrahydro 1H-cyclohept [c,d] indol-6-ol ;

— le 5-R,S-trans 5-méthylamino 3,4,5,6-tétrahydro 1H-cyclohept [c,d] indol-6-ol

ainsi que leurs sels d'addition avec les acides pharmaceutiquement acceptables.

En raison de leurs propriétés, les médicaments, selon la présente invention, peuvent être utilisés

dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet âgé, de l'athérosclérose et dans le traitement de l'hypertension d'origine rénale.

La dose usuelle, variable selon le produit utilisé, le sujet traité et l'affection en cause, peut être par exemple, de 5 mg à 200 mg par jour, par voie orale, chez l'homme, du produit de l'exemple 1.

L'invention a également pour objet les compositions pharmaceutiques qui renferment au moins un dérivé précité, ou l'un de ses sels d'addition avec les acides pharmaceutiquement acceptables, à titre de principe actif.

A titre de médicaments, les dérivés répondant à la formule I et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants dispersants ou émulsifiants, les conservateurs.

L'invention a également pour objet un procédé de préparation des dérivés, tels que définis par la formule I ci-dessus, ainsi que de leurs sels d'addition avec les acides, caractérisé en ce que l'on soumet un produit de formule II :

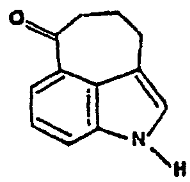

(II)

à l'action d'un halogénure de formule III

Hal—R' (III)

dans laquelle Hal représente un atome de chlore, de brome ou d'iode et R' a la signification de R, à l'exception d'hydrogène, pour obtenir un produit de formule IV :

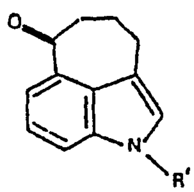

(IV)

puis soumet le produit de formule II ou IV, à l'action du nitrite de tert-butyle, en présence d'acide chlorhydrique, pour obtenir un produit de formule V :

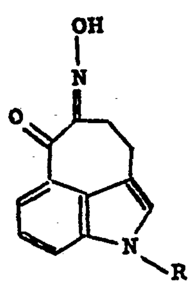

(V)

que l'on réduit pour obtenir un produit de formule $I_A$ :

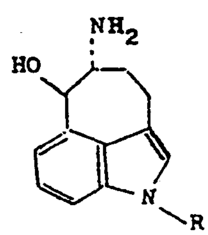

($I_A$)

dans laquelle R a la signification déjà indiquée, correspondant à un produit de formule I dans laquelle R est défini comme précédemment et $R_1$ représente un atome d'hydrogène, puis :

— soit l'on isole et, si désiré, salifie le produit de formule $I_A$,

— soit l'on traite le produit de formule $I_A$ par l'anhydride trifluoroacétique, de préférence en présence d'une base, pour obtenir un produit de formule VI :

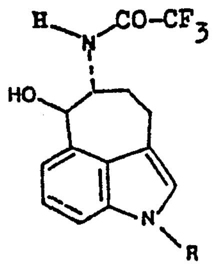

(VI)

dans laquelle R a la signification déjà indiquée, que l'on fait réagir avec un halogénure de formule VII :

$$Hal—R'_1 \quad (VII)$$

dans laquelle Hal représente un atome de chlore, de brome ou de préférence d'iode et $R'_1$ a la signification de $R_1$ déjà indiquée, à l'exception de l'hydrogène, pour obtenir un produit de formule VIII :

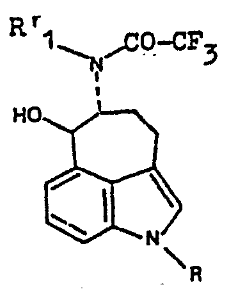

(VIII)

dans laquelle R et $R'_1$ ont la signification déjà indiquée, que l'on soumet à un agent capable d'hydrolyser l'amide trifluoroacétique pour obtenir un produit de formule $I_B$

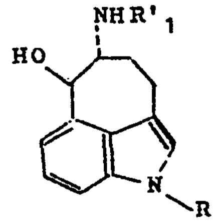

($I_B$)

dans laquelle R et $R'_1$ ont la signification déjà indiquée, correspondant à un produit de formule I dans laquelle $R_1$ a la signification de $R'_1$, produit de formule $I_B$, que l'on isole et, si désiré, salifie ;

— soit l'on traite le produit de formule $I_A$ par un haloformiate d'alcoyle, pour obtenir un produit de formule IX :

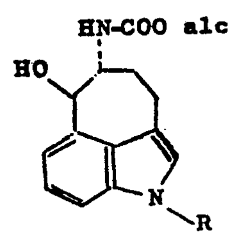

(IX)

dans laquelle R est défini comme précedemment et alc représente un radical alcoyle renfermant de 1 à 4 atomes de carbone, que l'on réduit pour obtenir un produit de formule $I_C$ :

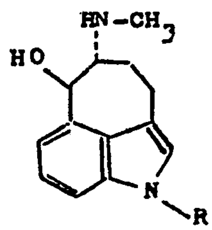

($I_C$)

dans laquelle R est défini comme précédemment, correspondant à un produit de formule I dans laquelle $R_1$ représente un radical méthyl, produit de formule $I_C$ que l'on isole et, si désiré, salifie,

— soit l'on traite le produit de formule $I_A$ par un agent d'acétylation, pour obtenir un produit de formule X :

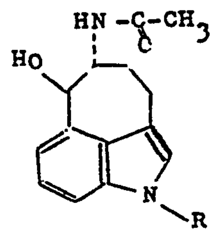

(X)

dans laquelle R est défini comme précédemment, que l'on réduit pour obtenir un produit de formule $I_D$ :

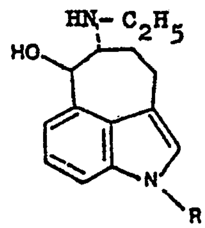

($I_D$)

dans laquelle R est défini comme précédemment, correspondant à un produit de formule I dans laquelle $R_1$ représente un radical éthyl, produit de formule $I_D$ que l'on isole et, si désiré, salifie ;

— soit l'on traite le produit de formule $I_A$ par l'acétone en milieu réducteur pour obtenir un produit de formule $I_E$ :

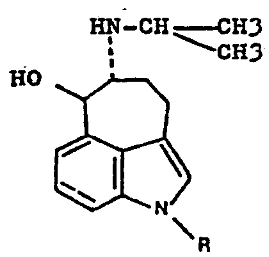

($I_E$)

dans laquelle R est défini comme précédemment, correspondant à un produit de formule I, dans laquelle $R_1$ représente un radical isopropyl, produit de formule $I_E$ que l'on isole et, si désiré, salifie.

Dans des conditions préférentielles d'exécution du procédé ci-dessus :

— la réaction entre les produits de formule II et III est effectuée par transfert de phase, selon la technique de A. Bosco et Al, décrite dans Synthésis *2,* 124 (1976), par exemple, en présence d'un sel d'ammonium quaternaire, tel que le chlorure de tétrabutyl ammonium, le chlorure de benzyltriphényl-phosphonium, ou de préférence l'hydrogènosulfate de tétrabutyl ammonium, de soude aqueuse et de benzène ;

— la réduction du produit de formule V est effectuée par hydrogénation catalytique suivie de l'action du borohydrure de sodium ;

— la base en présence de laquelle l'on traite le produit de formule $I_A$ par l'anhydride trifluoroacétique est une base minérale ;

— la réaction entre les produits de formule VI et VII est effectuée en présence d'un agent basique, tel qu'un carbonate ou un hydroxyde alcalin, ou une amine tertiaire ;

— l'hydrolyse du produit de formule VIII est effectuée par l'hydroxyde de sodium ou de potassium ;

— la réaction entre le produit de formule $I_A$ et l'haloformiate d'alcoyle est effectuée en présence d'une base telle qu'un carbonate alcalin ;

— la réduction des produits de formule IX et X est effectuée à l'aide d'hydrure d'aluminium-lithium ;

— l'agent d'acétylation que l'on fait réagir avec le produit de formule $I_A$ est un bromure ou un chlorure d'acétyle ou l'anhydride acétique ;

— l'agent réducteur que l'on fait agir avec l'acétone sur le produit de formule $I_A$ est, par exemple, le cyanoborohydrure de sodium.

Les sels des dérivés du cycloheptindolol répondant à la formule générale I, peuvent être préparés en faisant réagir en proportions sensiblement stœchiométriques, un acide minéral ou organique avec lesdits dérivés.

Les exemples suivants illustrent l'invention sans toutefois la limiter.

Exemple 1

5-R,S-trans amino 3,4,5,6-tétrahydro 1H-cyclohept [c,d] indol-6-ol et son acétate.

Stade A : 5-oxime de 3,4-dihydro 1H-cyclohept [c,d] indol 5,6-dione.

On dissout sous agitation et azote 18,5 g de 3,4-dihydro 1H-cyclohept [c,d] indol-6-one décrite dans Chem. Pharm. Bull. *25* (11) p. 3023, *1977*, dans 185 $cm^3$ de tétrahydrofuranne, refroidit au bain de glace, ajoute 22 $cm^3$ d'éthanol chlorhydrique à 5 moles/l, puis 13,5 $cm^3$ de nitrite de terbutyle, agite encore pendant 30 minutes au bain de glace, essore, lave à l'éther, sèche sous pression réduite à 80 °C et obtient 19,4 g de cristaux fondant avec décomposition à 235 °C.

Stade B : 5-R,S-trans amino 3,4,5,6-tétrahydro 1H-cyclohept [c,d] indol-6-ol et son acétate.

On met en suspension 5 g du produit obtenu au stade précédent dans 100 $cm^3$ de méthanol, ajoute 2, 5 g de palladium sur charbon actif à 10 %, agite pendant 40 minutes à température ambiante sous hydrogène, filtre, refroidit le filtrat au bain de glace, ajoute 2,5 g de borohydrure de sodium en 10 minutes, laisse revenir à température ambiante sous atmosphère inerte, agite pendant 1 heure, évapore à sec, reprend au chlorure de méthylène au reflux, filtre, distille à sec, redissout à 50 °C dans 7 $cm^3$ de méthanol, ajoute 70 $cm^3$ d'acétate d'éthyle, filtre, rince à l'acétate d'éthyle, concentre à 50 $cm^3$, ajoute alors 25 $cm^3$ de solution d'acide acétique dans l'acétate d'éthyle 1,1 N, laisse reposer à température ambiante pendant 1 heure, glace pendant 30 minutes, essore, sèche sous pression réduite à 70 °C, et récupère 4,9 g de l'acétate du produit attendu fondant à 150 °C. Par recristallisation dans le mélange méthanol-acétate d'éthyle 1-1, on obtient l'acétate sous forme d'un solide blanc cristallisé fondant à 165 °C.

Analyse de l'acétate : $C_{12}H_{14}N_2O$, $C_2H_4O_2$ = 262,30

Calculé : C% 64,10 H% 6,92 N% 10,68

Trouvé : C% 64,2 H% 7,1 N%10,4

Libération de la base : On met en suspension 10,8 g d'acétate tel qu'obtenu ci-dessus dans 300 $cm^3$ d'acétate d'éthyle, ajoute sous agitation 100 $cm^3$ d'ammoniaque concentrée, sature de chlorure de sodium, agite sous azote pendant 15 minutes, décante, lave à l'eau salée, sèche, amène à sec et obtient 9, 8 g du produit attendu.

Exemple 2

Chlorhydrate de R,S-trans 5-méthylamino 3,4,5,6-tétrahydro 1H-cyclohept [c,d] indol-6-ol.

Stade A : R,S-trans N-(6-hydroxy 3,4,5,6-tétrahydro 1H-cyclohept [c,d] indol-5-yl) carbamate d'éthyle.

On maintient sous agitation 2,62 g d'acétate de 5-R -trans amino 3,4,5,6-tétrahydro 1H-cyclohept [c,d] indol-6-ol, 52 $cm^3$ d'acétate d'éthyle, 40 $cm^3$ d'eau et 2,62 g de carbonate de potassium, ajoute en 10 minutes à température ambiante 2,6 $cm^3$ de chloroformiate d'éthyle, agite pendant 1 heure, distille sous pression réduite, dilue avec un peu d'eau glacée, triture pendant 30 minutes à température ambiante, essore et sèche sous pression réduite à 60-70 °C. On introduit le produit dans du méthanol, agite, filtre, concentre, amorce la cristallisation, glace pendant 3 heures et récupère 1,45 g de cristaux fondant à 190 °C.

Stade B : Chlorhydrate de R,S-trans 5-méthylamino 3,4,5,6-tétrahydro 1H-cyclohept [c,d] indol-6-ol.

On met en suspension à température ambiante sous atmosphère inerte et sous agitation 3,6 g d'hydrure d'aluminium lithium dans 50 $cm^3$ de tétrahydrofuranne, ajoute en 20 minutes une solution de 1, 83 g de carbamate tel qu'obtenu ci-dessus, dans 75 $cm^3$ de dioxanne. On porte au reflux pendant

2 heures, refroidit, ajoute en 30 minutes, 50 $cm^3$ de tétrahydrofuranne hydraté à 10 %, dilue par 50 $cm^3$ d'eau, filtre, rince le filtre à l'acétate d'éthyle, ajoute du chlorure de sodium au filtrat, décante, extrait à l'acétate d'éthyle, lave à l'eau puis à l'aide d'une solution aqueuse saturée de chlorure de sodium, sèche sur sulfate de sodium distille à sec, et récupère 2 g de produit brut.

Préparation du chlorydrate :

On dissout le produit obtenu ci-dessus, dans 3 $cm^3$ d'éthanol, ajoute goutte à goutte 1,5 $cm^3$ d'éthanol chlorhydrique 5 N, glace pendant 1 heure, essore, lave à l'éthanol, sèche sous pression réduite à 70 °C et obtient 1,24 g de cristaux fondant à 224 °C avec décomposition.

Analyse : $C_{13}H_{16}N_2O$, HCl = 252,7

Calculé : C% 61,78 H% 6,78 N% 11,08 C1% 14,03

Trouvé : C% 61,8 H% 6,7 N% 11,1 C1% 13,9

Exemple 3

R,S-trans 5-(1-méthyléthyl) amino 3,4,5,6-tétrahydro 1H-cyclohept [c,d] indol-6-ol.

On met en suspension sous agitation et atmosphère inerte 10,5 g d'acétate de R,S-trans 5-amino 3,4,5,6-tétrahydro 1H-cyclohept [c,d] indol-6-ol dans 105 $cm^3$ de méthanol et 52,5 $cm^3$ d'acétone, refroidit vers 0 °C, ajoute en 15 minutes 10,5 g de cyanoborohydrure de sodium, agite pendant 3 heures 30 minutes vers 0 °C, filtre, rince à l'acétate d'éthyle, évapore à sec, chromatographie sur colonne de silice sous pression (éluant benzène-acétate d'éthyle-triéthylamine 7-2-1) et récupère 6,32 g de cristaux. On redissout les cristaux dans 6 $cm^3$ d'acétate d'éthyle à chaud, laisse refroidir pendant 1 heure 30 minutes, essore, sèche sous pression réduite à température ambiante et obtient 4,03 g de cristaux blancs fondant à 100 °C.

Analyse : $C_{15}H_{20}N_2O$ = 244,338

Calculé : C% 73,74 H% 8,25 N% 11,46

Trouvé : C% 73,5 H% 8,3 N% 11,4

Exemple 4

Acétate de 5-R,S-trans 5-amino 1-méthyl 3,4,5,6-tétrahydro 1H-cyclohept [c,d] indol-6-ol.

Stade A : 1-méthyl 1,3,4,5-tétrahydro 6H-cyclohept [c,d] indol-6-one.

On agite pendant 30 minutes à 40-45 °C sous atmosphère inerte, 5 g de 3,4-dihydro 1H-cyclohept [c,d] indol-6-one avec 50 $cm^3$ de benzène, 2,5 $cm^3$ d'iodure de méthyle, 25 $cm^3$ de soude 5N et 9,2 g d'hydrogènosulfate de tétra n-butylammonium, dilue à l'eau, décante, extrait à l'acétate d'éthyle, lave à l'acide chlorhydrique N, à l'eau, sèche la phase organique, amène à sec, purifie le résidu par chromatographie sur silice (éluant : chloroforme-acétate d'éthyle 95-5), récupère 6,3 g de produit brut, que l'on cristallise dans l'isopropanol et obtient 5,15 g du produit attendu (p.f. ≃ 70 °C).

Stade B : 5-oxime de 1-méthyl 3,4-dihydro 1H-cyclohept [c,d] indol 5,6-dione.

On dissout sous atmosphère inerte 10,41 g de produit tel qu'obtenu ci-dessus, dans 20 $cm^3$ de tétrahydrofuranne, ajoute 85 $cm^3$ d'éther éthylique, refroidit à − 5 °C, ajoute 16,4 $cm^3$ d'éthanol chlorhydrique 3,5 N, agite pendant 5 minutes, introduit en 30 minutes 7 $cm^3$ de nitrite de tert-butyle, agite pendant 30 minutes en maintenant la température en dessous de 0 °C, essore, lave à l'éther éthylique, sèche sous pression réduite et obtient 10,5 g du produit attendu (p. f. = 190 °C puis 200 °C).

Stade C : Acétate de 5 R,S-trans 5-amino 1-méthyl 3,4,5,6-tétrahydro 1H-cyclohept [c,d] indol-6-ol.

Pendant 1 heure 45 minutes on hydrogène les 10,5 g obtenus ci-dessus, dans 210 $cm^3$ de méthanol, avec 5,25 g de charbon palladié à 10 %, filtre, refroidit au bain de glace, ajoute 5,25 g de borohydrure de sodium en 10 minutes, sous atmosphère inerte, agite encore 10 minutes, puis 40 minutes à température ambiante, distille le méthanol sous pression réduite, ajoute 30 $cm^3$ d'eau, extrait à l'acétate d'éthyle, lave à l'eau, sèche, distille à sec sous pression réduite, purifie par chromatographie sur silice (éluant : acétate d'éthyle-méthanol-ammoniaque 90-5-5) et obtient 9,1 g de base du produit attendu.

Préparation de l'acétate :

On dissout la base ci-dessus dans 10 $cm^3$ d'éthanol, ajoute lentement de l'acide acétique dans l'acétate d'éthyle, en léger excès, laisse pendant 48 heures au repos, essore, lave à l'éthanol, à l'acétate d'éthyle, à l'éther éthylique, sèche et obtient 4,12 g d'acétate (p. f. ≃ 140 °C).

Après recristallisation dans l'isopropanol (p. f. : ≃ 140 °C puis 160 °C).

Analyse : $C_{15}H_{20}N_2O_3$ = 276, 336

Calculé : C % 65,19 H % 7,29 N % 10,13

Trouvé : C % 65,2 H % 7,4 N % 10,2

Exemple 5

On a préparé des comprimés répondant à la formule :

— Acétate de 5-R,S-trans amino 3,4,5,6-tétrahydro 1H-cyclohept [c,d] indol-6-ol 10 mg ;
— Excipient q. s. pour un comprimé terminé à 100 mg.

(Détail de l'excipient : lactose, amidon, talc, stéarate de magnésium).

Exemple 6

On a préparé des comprimés répondant à la formule :

— Chlorhydrate de R,S-trans 5-méthylamine 3,4,5,6-tétrahydro 1H-cyclohept [c,d] indol-6-ol 15 mg ;
— Excipient q. s. pour un comprimé terminé à 100 mg.

(Détail de l'excipient : lactose, amidon, talc, stéarate de magnésium).

Etude pharmacologique :

A) Action antihypertensive chez le chien éveillé.

L'étude de l'action antihypertensive du produit de l'exemple 1 a été effectué sur le chien Beagle pesant de 12 kg à 14 kg rendu hypertendu par enveloppement de 2 reins par de la cellophane selon la technique décrite par Irvine H. Page dans Science (1939) *89* p. 273-274.

Le produit a été administré per os aux doses de 1 mg/kg et 10 mg/kg.

La pression artérielle a été mesurée à la queue du chien au moyen d'un manchon pneumatique et à l'aide d'un transducteur piezo-électrique de pression. La pression a été mesurée avant et 1 heure, 3 heures, 6 heures et 24 heures après administration du produit.

Le tableau ci-après indique que les variations exprimées en pourcentages de la pression artérielle, après administration du produit, par rapport à la pression témoin initiale.

| Produit de l'exemple | Dose mg/kg | Variation % de la pression artérielle | | | |
|---|---|---|---|---|---|
| | | 1er jour | | | |
| | | 1 heure après l'adminis-tration | 3 heures après l'adminis-tration | 6 heures après l'adminis-tration | 24 heures après l'administration |
| 1 | 1 | – 16 | – 17 | – 13 | – 10 |
| | 10 | – 11 | – 27 | – 10 | – 32 |

B) Détermination de l'activité antihypertensive.

L'action antihypertensive a été étudiée sur des rats mâles spontanément hypertendus (souche OKAMOTO) âgés de 20 semaines pesant 300 à 320 g.

Le produit a été administré par voie orale 48 heures après la pose d'un cathéter intracarotidien.

La pression artérielle a été mesurée à la queue du rat au moyen d'un manchon pneumatique et à l'aide d'un transducteur piezo-électrique de pression. La pression a été mesurée avant et 1 heure, 4 heures et 24 heures après l'administration du produit.

Le tableau ci-après indique les variations exprimées en pourcentages de la pression artérielle, après administration du produit, par rapport à la pression témoin initiale.

| Produit de l'exemple | Dose mg/kg | Variation % de la pression artérielle | | |
|---|---|---|---|---|
| | | 1 heure après l'administration | 4 heures après l'administration | 24 heures après l'administration |
| 1 | 1 | - 24 | - 21 | - 12 |

C) Détermination de l'activité hypotensive.

L'activité hypotensive a été étudiée sur des rats mâles de souche WISTAR pesant 300 g environ et anesthésiés au nembutal (50 mg/kg par voie intraveineuse).

Le produit testé a été administré par voie intraveineuse dans la veine jugulaire.

La pression artérielle carotidienne a été mesurée avant et après administration du produit testé.

Le tableau ci-après indique les variations exprimées en pourcentage de la pression artérielle après administration du produit testé par rapport à la pression artérielle témion initiale.

| Produits de l'exemple | Dose mg/kg | Variation % de la pression artérielle | | | |
|---|---|---|---|---|---|
| | | 1 minute après l'administration | 5 minutes après l'administration | 10 minutes après l'administration | 30 minutes après l'administration |
| 1 | 0,1 | - 3 | - 15 | - 28 | - 34 |
| | 0,01 | 0 | - 7 | - 16 | - 16 |
| 2 | 1 | - 11 | - 28 | - 34 | - 30 |
| | 0,1 | - 1 | - 12 | - 17 | - 10 |
| 3 | 10 | - 34 | - 31 | - 26 | - 29 |
| | 1 | - 15 | - 18 | - 18 | - 21 |
| 4 | 10 | - 31 | - 21 | - 15 | 0 |

D) Etude de la toxicité aiguë.

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants :

| Produits de l'exemple | $DL_0$ en mg/kg |
|---|---|
| 1 | 200 |
| 2 | 200 |
| 3 | 100 |
| 4 | 200 |

**Revendications** (pour les Etats contractants : BE, CH, DE, FR, GB, IT, LI, LU, NL, SE)

1. Les dérivés du cycloheptindolol répondant à la formule générale I :

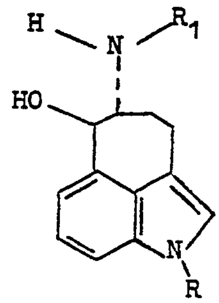

(I)

dans laquelle R représente un atome d'hydrogène, un radical alcoyle renfermant de 1 à 5 atomes de carbone ou un radical aralcoyle renfermant de 7 à 12 atomes de carbone, éventuellement substitué par des radicaux chloro, bromo, méthyl, éthyl, méthoxy, trifluorométhyl ou méthylthio, et $R_1$ représente un atome d'hydrogène, un radical alcoyle renfermant de 1 à 5 atomes de carbone, cycloalcoylalcoyle renfermant de 4 à 7 atomes de carbone, ou aralcoyle renfermant de 7 à 12 atomes de carbone, éventuellement substitué par des radicaux chloro, bromo, méthyl, éthyl, méthoxy, trifluorométhyl ou méthylthio ainsi que les sels d'addition avec les acides minéraux ou organiques desdits dérivés.

2. Les dérivés du cycloheptindole répondant à la formule I de la revendication 1, dans laquelle R et $R_1$ représentent un atome d'hydrogène ou un radical alcoyle renfermant de 1 à 5 atomes de carbone, ainsi que les sels d'addition avec les acides minéraux ou organiques desdits dérivés.

3. Le 5R,S-trans amino 3,4,5,6-tétrahydro 1H-cyclohept [c,d] indol-6-ol ainsi que ses sels d'addition avec les acides minéraux ou organiques.

4. Le 5R,S-trans 5-méthylamino 3,4,5,6-tétrahydro 1H-cyclohept [c,d] indol-6-ol, ainsi que ses sels d'addition avec les acides minéraux ou organiques.

5. Procédé de préparation des dérivés de formule I, telle que définie à la revendication 1, ainsi que de leurs sels d'addition avec les acides minéraux ou organiques, caractérisé en ce que l'on soumet un produit de formule II :

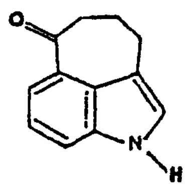

(II)

à l'action d'un halogénure de formule III

Hal—R' (III)

dans laquelle Hal représente un atome de chlore, de brome ou d'iode et R' à la signification de R, à l'exception d'hydrogène ; pour obtenir un produit de formule IV :

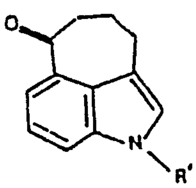

(IV)

puis soumet le produit de formule II ou IV, à l'action du nitrite de tert-butyle, en présence d'acide chlorhydrique, pour obtenir un produit de formule V :

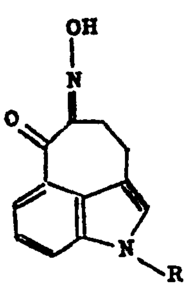

(V)

que l'on réduit pour obtenir un produit de formule $I_A$ :

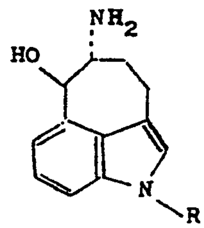

($I_A$)

dans laquelle R a la signification déjà indiquée, correspondant à un produit de formule I dans laquelle R est défini comme précédemment et $R_1$ représente un atome d'hydrogène, puis :

— soit l'on isole et, si désiré, salifie le produit de formule $I_A$,

— soit l'on traite le produit de formule $I_A$ par l'anhydride trifluoroacétique, de préférence en présence d'une base, pour obtenir un produit de formule VI :

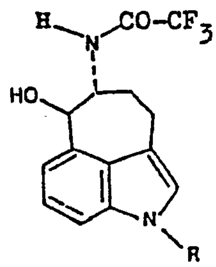

(VI)

dans laquelle R a la signification déjà indiquée, que l'on fait réagir avec un halogénure de formule VII :

$$Hal—R'_1 \qquad (VII)$$

dans laquelle Hal représente un atome de chlore, de brome ou de préférence d'iode et $R'_1$ a la signification de $R_1$ déjà indiquée, à l'exception de l'hydrogène, pour obtenir un produit de formule VIII :

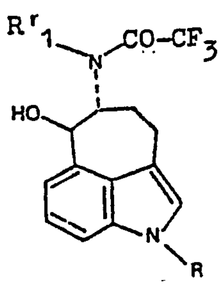

(VIII)

dans laquelle R et $R'_1$ ont la signification déjà indiquée, que l'on soumet à un agent capable d'hydrolyser l'amide trifluoroacétique pour obtenir un produit de formule $I_B$

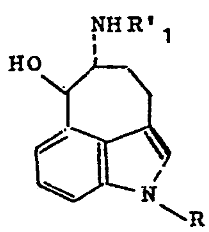

($I_B$)

dans laquelle R et $R'_1$ ont la signification déjà indiquée, correspondant à un produit de formule I dans laquelle $R_1$ a la signification de $R'_1$, produit de formule $I_B$, que l'on isole et, si désiré, salifie ;

— soit l'on traite le produit de formule $I_A$ par un haloformiate d'alcoyle, pour obtenir un produit de formule IX :

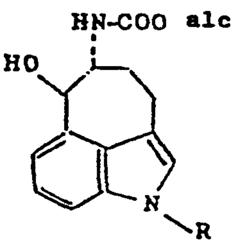

(IX)

dans laquelle R est défini comme précédemment et alc représente un radical alcoyle renfermant de 1 à 4 atomes de carbone, que l'on réduit, pour obtenir un produit de formule $I_C$ :

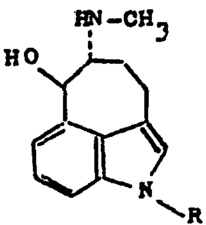

($I_C$)

dans laquelle R est défini comme précédemment, correspondant à un produit de formule I dans laquelle $R_1$ représente un radical méthyl, produit de formule $I_c$ que l'on isole et, si désiré, salifie,

— soit l'on traite le produit de formule $I_A$ par un agent d'acétylation, pour obtenir un produit de formule X :

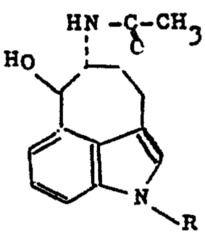

(X)

dans laquelle R est défini comme précédemment, que l'on réduit pour obtenir un produit de formule $I_D$ :

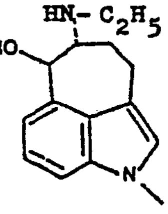

($I_D$)

dans laquelle R est défini comme précédemment, correspondant à un produit de formule I dans laquelle $R_1$ représente un radical éthyl, produit de formule $I_D$ que l'on isole et, si désiré, salifie ;

— soit l'on traite le produit de formule $I_A$ par l'acétone en milieu réducteur pour obtenir un produit de formule $I_E$ :

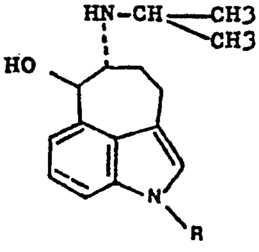

($I_E$)

dans laquelle R est défini comme précédemment, correspondant à un produit de formule I, dans laquelle $R_1$ représente un radical isopropyle, produit de formule $I_E$ que l'on isole et, si désiré, salifie.

6. Médicaments, caractérisés en ce qu'ils sont constitués par les dérivés du cycloheptindolol, tels que définis par la formule I de la revendication 1, ainsi que par leurs sels d'addition avec les acides minéraux ou organiques pharmaceutiquement acceptables.

7. Médicaments, caractérisés en ce qu'ils sont constitués par les dérivés du cycloheptindolol, tels que définis à la revendication 2, ainsi que par leurs sels d'addition avec les acides minéraux ou organiques pharmaceutiquement acceptables.

8. Médicaments, caractérisés en ce qu'ils sont constitués par les dérivés du cycloheptindolol, tels que définis à la revendication 3 ou 4, ainsi que par leurs sels d'addition avec les acides pharmaceutiquement acceptables.

9. Compositions pharmaceutiques, caractérisées en ce qu'elles renferment, à titre de principe actif, l'un ou moins des médicaments définis dans l'une des revendications 6, 7 ou 8.

**Revendications** (pour l'Etat Contractant AT)

1. Procédé pour préparer les dérivés du cycloheptindolol répondant à la formule générale I :

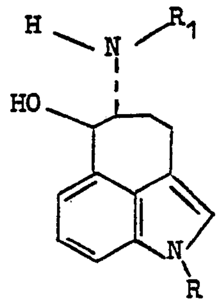

(I)

dans laquelle R représente un atome d'hydrogène, un radical alcoyle renfermant de 1 à 5 atomes de carbone ou un radical aralcoyle renfermant de 7 à 12 atomes de carbone, éventuellement substitué par des radicaux chloro, bromo, méthyl, éthyl, méthoxy, trifluorométhyl ou méthylthio, et $R_1$ représente un atome d'hydrogène, un radical alcoyle renfermant de 1 à 5 atomes de carbone, cycloalcoylalcoyle renfermant de 4 à 7 atomes de carbone ou aralcoyle renfermant de 7 à 12 atomes de carbone, éventuellement substitué par des radicaux chloro, bromo, méthyl, éthyl, méthoxy, trifluorométhyl ou méthylthio, ainsi que les sels d'addition avec les acides minéraux ou organiques, caractérisé en ce que l'on soumet un produit de formule II :

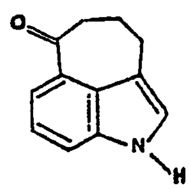

(II)

à l'action d'un halogénure de formule III :

$$Hal—R' \qquad (III)$$

dans laquelle Hal représente un atome de chlore, de brome ou d'iode et R' a la signification de R, à l'exception d'hydrogène pour obtenir un produit de formule IV :

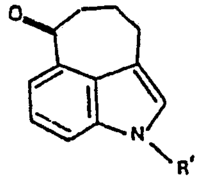

(IV)

puis soumet le produit de formule II ou IV, à l'action du nitrite de tert-butyle, en présence d'acide chlorhydrique, pour obtenir un produit de formule V :

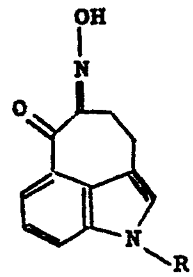

(V)

que l'on réduit pour obtenir un produit de formule $I_A$ :

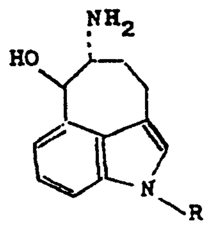

($I_A$)

dans laquelle R a la signification déjà indiquée, correspondant à un produit de formule I dans laquelle R est défini comme précédemment et $R_1$ représente un atome d'hydrogène, puis :

— soit l'on isole et, si désiré, salifie le produit de formule $I_A$,

— soit l'on traite le produit de formule $I_A$ par l'anhydride trifluoroacétique, de préférence en présence d'une base, pour obtenir un produit de formule VI :

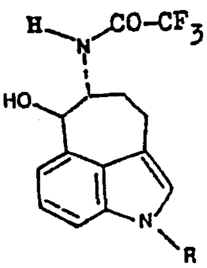

(VI)

dans laquelle R a la signification déjà indiquée, que l'on fait réagir avec un halogénure de formule VII :

Hal—$R'_1$ (VII)

dans laquelle Hal représente un atome de chlore, de brome ou de préférence d'iode et $R'_1$ a la signification de $R_1$ déjà indiquée, à l'exception de l'hydrogène, pour obtenir un produit de formule VIII :

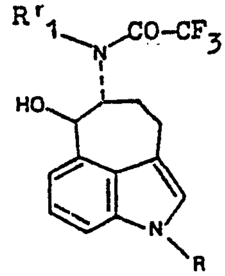

(VIII)

dans laquelle R et $R'_1$ ont la signification déjà indiquée, que l'on soumet à un agent capable d'hydrolyser l'amide trifluoroacétique pour obtenir un produit de formule $I_B$ :

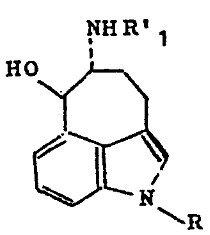

($I_B$)

dans laquelle R et $R'_1$ ont la signification déjà indiquée, correspondant à un produit de formule I dans laquelle $R_1$ a la signification de $R'_1$, produit de formule $I_B$, que l'on isole et, si désiré, salifie :

— soit l'on traite le produit de formule $I_A$ par un haloformiate d'alcoyle, pour obtenir un produit de formule IX :

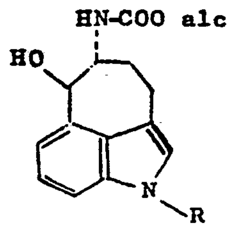

(IX)

dans laquelle R est défini comme précédemment et alc représente un radical alcoyle renfermant de 1 à 4 atomes de carbone, que l'on réduit, pour obtenir un produit de formule $I_C$ :

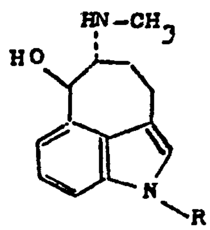

($I_C$)

dans laquelle R est défini comme précédemment, correspondant à un produit de formule I dans laquelle $R_1$ représente un radical méthyl, produit de formule $I_C$ que l'on isole et, si désiré, salifie,

— soit l'on traite le produit de formule $I_A$ par un agent d'acétylation, pour obtenir un produit de formule X :

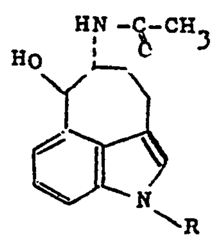

(X)

dans laquelle R est définit comme précédemment, que l'on réduit pour obtenir un produit de formule $I_D$ :

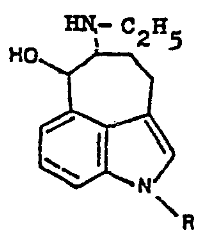

($I_D$)

dans laquelle R est défini comme précédemment, correspondant à un produit de formule I dans laquelle $R_1$ représente un radical éthyl, produit de formule $I_D$ que l'on isole et, si désiré, salifie ;

— soit l'on traite le produit de formule $I_A$ par l'acétone en milieu réducteur, pour obtenir un produit de formule $I_E$ :

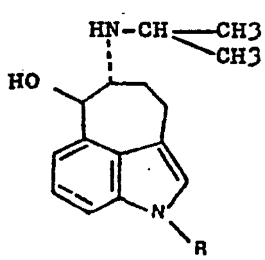

($I_E$)

dans laquelle R est défini comme précédemment, correspondant à un produit de formule I, dans laquelle $R_1$ représente un radical isopropyle, produit de formule $I_E$ que l'on isole et, si désiré, salifie.

2. Procédé selon la revendication 1, caractérisé en ce que l'on utilise au départ un halogénure de formule III dans lequel R' représente un radical alcoyle renfermant de 1 à 5 atomes de carbone.

3. Procédé selon la revendication 1 ou 2, caractérisé en ce que l'on utilise au départ un halogénure de formule VII dans lequel $R'_1$ représente un radical alcoyle renfermant de 1 à 5 atomes de carbone.

4. Procédé selon la revendication 1, caractérisé en ce que l'on prépare le 5-R,S-trans amino 3,4,5,6-tétrahydro 1H-cyclohept [c,d] indol-6-ol, ainsi que ses sels d'addition avec les acides.

5. Procédé selon la revendication 1, caractérisé en ce que l'on prépare le 5-R,S-trans 5-méthylamino 3,4,5,6-tétrahydro 1H-cyclohept [c,d] indol-6-ol ainsi que ses sels d'addition avec les acides.

**Claims** (for the Contracting States : BE, CH, DE, FR, GB, IT, LI, LU, NL, SE)

1. Derivatives of cycloheptindolol corresponding to the general formula I :

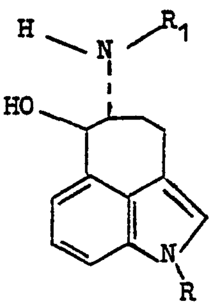

(I)

in which R represents a hydrogen atom, an alkyl radical containing from 1 to 5 carbon atoms, or an aralkyl radical containing from 7 to 12 carbon atoms, possibly substituted by chlorine, bromine, methyl, ethyl, methoxy, trifluoromethyl, or methylthio radicals, and $R_1$ represents a hydrogen atom, an alkyl radical containing from 1 to 5 carbon atoms, a cycloalkylalkyl radical containing from 4 to 7 carbon atoms, or an aralkyl radical containing from 7 to 12 carbon atoms, possibly substituted by chlorine, bromine, methyl, ethyl, methoxy, trifluoromethyl or methylthio radicals, as well as the salts of addition with mineral or organic acids of the said derivatives.

2. The derivatives of cycloheptindole corresponding to the formula I of claim 1, in which R and $R_1$ represent a hydrogen atom or an alkyl radical containing from 1 to 5 carbon atoms, as well as the salts of addition with mineral or organic acids of the said derivatives.

3. 5R,S-trans amino 3,4,5,6-tetrahydro 1H-cyclohept [c,d] indol-6-ol as well as its salts of addition with mineral or organic acids.

4. 5R,S-trans 5-methylamino 3,4,5,6-tetrahydro 1H-cyclohept [c,d] indol-6-ol, as well as its salts of addition with mineral or organic acids.

5. Preparation process for the derivatives with the formula I, as defined in claim 1, as well as their salts of addition with mineral or organic acids, characterised in that a product with the formula II :

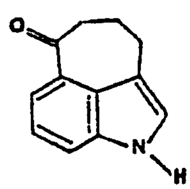

(II)

is submitted to the action of a halide with the formula III :

$$Hal—R' \quad (III)$$

in which Hal represents a chlorine, bromine or iodine atom, and R' has the significance of R, with the exception of hydrogen, so as to obtain the product with the formula IV :

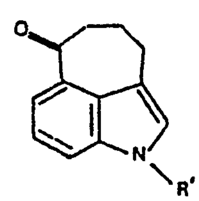

(IV)

Then the product with the formula II or IV, is submitted to the action of tert-butyl nitrite, in the presence of hydrochloric acid, so as to obtain the product with the formula V :

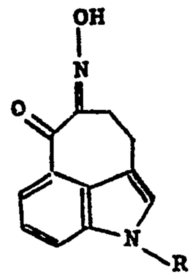

(V)

which is reduced so as to obtain a product with the formula $I_A$ :

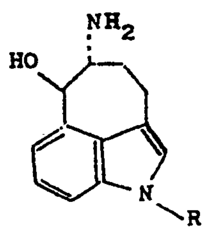

($I_A$)

in which R has the significance already indicated, corresponding to a product with the formula I in which R is defined as previously and $R_1$ represents a hydrogen atom, then :

— either the product with the formula $I_A$ is isolated and if desired salified,

— or the product with the formula $I_A$ is treated with trifluoroacetic anhydride, preferably in the presence of a base, so as to obtain a product with the formula VI :

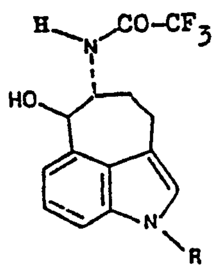

(VI)

in which R has the significance already indicated, which is made to react with a halide with the formula VII :

$$Hal—R'_1 \quad (VII)$$

in which Hal represents a chlorine, bromine or preferably an iodine atom, and $R'_1$ has the significance of $R_1$ as already indicated, with the exception of hydrogen, so as to obtain a product with the formula VIII :

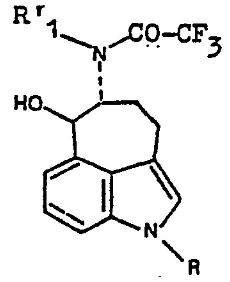

(VIII)

in which R and $R'_1$ have the already indicated significance, which is submitted to an agent able to hydrolize the trifluoroacetic amide so as to obtain a product with the formula $I_B$ :

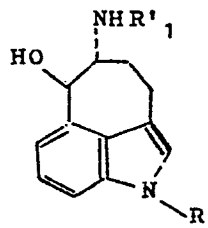

($I_B$)

in which R and $R'_1$ have the significances already indicated, corresponding to a product with the formula I in which $R_1$ has the significance of $R'_1$, which product with the formula $I_B$ is isolated and if desired salified ;

— or the product with the formula $I_A$ is treated by an alkyl haloformate, so as to obtain a product with the formula IX :

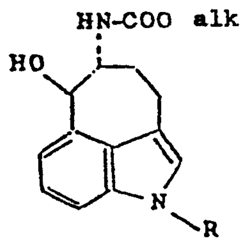

(IX)

in which R is defined as previously and alk represents an alkyl radical containing from 1 to 4 carbon atoms, which is reduced, so as to obtain a product with the formula $I_C$ :

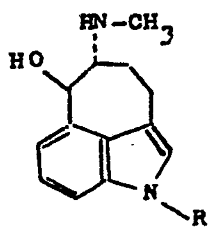

($I_C$)

in which R is defined as previously, corresponding to a product with the formula I in which $R_1$ represents a methyl radical, which product with the formula $I_C$ is isolated and if desired salified,

— or the product with the formula $I_A$ is treated by an acetylation agent so as to obtain the product with the formula X :

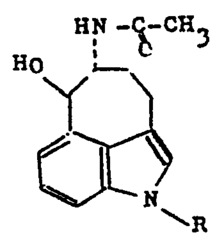

(X)

in which R is defined as previously, which is reduced so as to obtain the product with the formula $I_D$ :

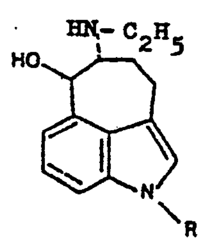

($I_D$)

in which R is defined as previously, corresponding to a product with the formula I in which $R_1$ represents an ethyl radical, which product with the formula $I_D$ is isolated and if desired salified ;

— or the product with the formula $I_A$ is treated by acetone in a reducing medium so as to obtain the product with the formula $I_E$ :

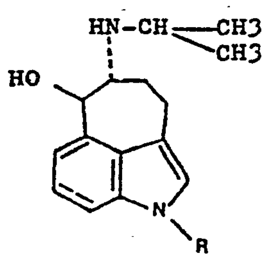

($I_E$)

in which R is defined as previously, corresponding to a product with the formula I, in which $R_1$ represents an isopropyl radical, which product with the formula $I_E$ is isolated and if desired salified.

6. Medicaments, characterised in that they are constituted by the derivatives of cycloheptindolol, as defined by the formula I of claim 1, as well as by their salts of addition with pharmaceutically acceptable mineral or organic acids.

7. Medicaments, characterised in that they are constituted by the derivatives of cycloheptindolol, as defined in claim 2, as well as by their salts of addition with pharmaceutically acceptable mineral or organic acids.

8. Medicaments, characterised in that they are constituted by the derivatives of cycloheptindolol, as defined in claim 3 or 4, as well as by their salts of addition with the pharmaceutically acceptable acids.

9. Pharmaceutical compositions, characterised in that they contain as active principle one at least of the medicaments defined in one of the claims 6, 7 or 8.

**Claims** (for the Contracting State AT)

1. Process for the preparation of the derivatives of cycloheptindolol corresponding to the general formula I :

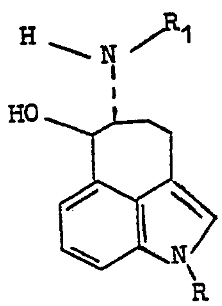

(I)

in which R represents a hydrogen atom, an alkyl radical containing from 1 to 5 carbon atoms, or an aralkyl radical containing from 7 to 12 carbon atoms, possibly substituted by chlorine, bromine, methyl, ethyl, methoxy, trifluoromethyl, or methylthio radicals, and $R_1$ represents a hydrogen atom, an alkyl radical containing from 1 to 5 carbon atoms, a cycloalkylalkyl radical containing from 4 to 7 carbon atoms or an aralkyl radical containing from to 7 to 12 carbon atoms, possibly substituted by chlorine, bromine, methyl, ethyl, methoxy, trifluoromethyl or methylthio radicals, as well as the salts of addition with mineral or organic acids, characterised in that a product with the formula II :

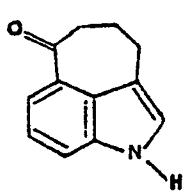

(II)

is submitted to the action of a halide with the formula III :

Hal—R′ (III)

in which Hal represents a chlorine, bromine or iodine atom and R′ has the significance of R, with the exception of hydrogen, so as to obtain the product with the formula IV :

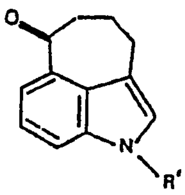

(IV)

then submit the product with the formula II or IV, to the action of tertbutyl nitrite, in the presence of hydrochloric acid, so as to obtain a product with the formula V :

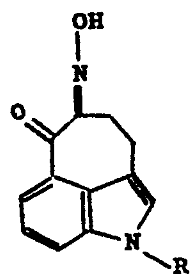

(V)

which is reduced so as to obtain the product with the formula $I_A$ :

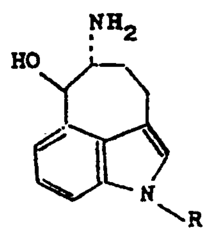

($I_A$)

in which R has the significance already indicated, corresponding to a product with the formula I in which R is defined as previously and $R_1$ represents a hydrogen atom, then :

— either the product with the formula $I_A$, is isolated and if desired salified,

— or the product with the formula $I_A$ is treated with trifluoroacetic anhydride, preferably in the presence of a base, so as to obtain the product with the formula VI :

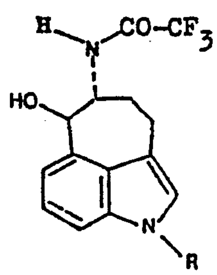

(VI)

in which R has the significance already indicated, which is made to react with a halide with the formula VII :

$$Hal{-}R'_1 \qquad (VII)$$

in which Hal represents a chlorine, bromine, or preferably an iodine atom and $R'_1$ has the significance of $R_1$ already indicated, with the exception of hydrogen, so as to obtain the product with the formula VIII :

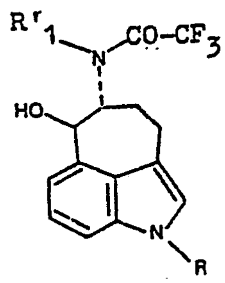

(VIII)

in which R and R'$_1$ have the significance already indicated, which is submitted to an agent able to hydrolize trifluoroacetic amide so as to obtain the product with the formula $I_B$ :

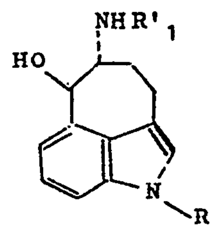

($I_B$)

in which R and R'$_1$ have the already indicated significance, corresponding to a product with the formula I in which $R_1$ has the significance of R'$_1$, which product with the formula $I_B$ is isolated and if desired salified ;

— or the product with the formula $I_A$ is treated with an alkyl haloformate so as to obtain the product with the formula IX :

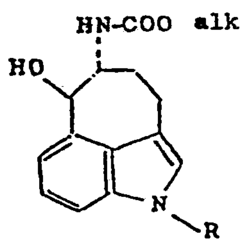

(IX)

in which R is defined as previously and alk represents an alkyl radical containing from 1 to 4 carbon atoms, which is reduced, so as to obtain the product with the formula $I_C$ :

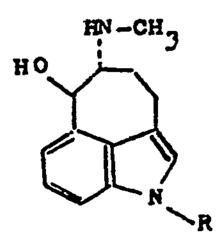

($I_C$)

in which R is defined as previously, corresponding to a product with the formula I in which $R_1$ represents a methyl radical, which product with the formula $I_C$ is isolated, and if desired salified,

— or the product with the formula $I_A$ is treated by an acetylation agent, so as to obtain the product with the formula X :

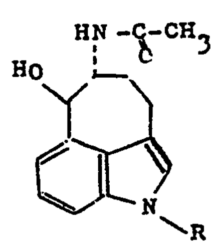

(X)

in which R is defined as previously, which is reduced so as to obtain the product with the formula $I_D$ :

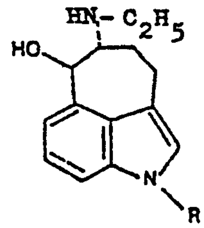

($I_D$)

in which R is defined as previously, corresponding to a product with the formula I in which $R_1$ represents an ethyl radical, which product with the formula $I_D$ is isolated and if desired salified ;

— or the product with the formula $I_A$ is treated with acetone in a reducing medium so as to obtain a product with the formula $I_E$ :

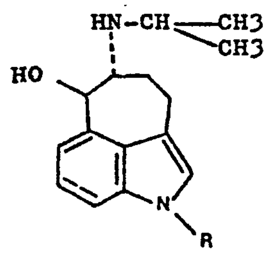

($I_E$)

in which R is defined as previously, corresponding to a product with the formula I, in which $R_1$ represents an isopropyl radical, which product with the formula $I_E$ is isolated and if desired salified.

2. Process according to claim 1, characterised in that at the start a halide with the formula III is used in which R' represents an alkyl radical having from 1 to 5 carbon atoms.

3. Process according to claim 1 or 2, characterised in that at the start a halide with the formula VII is used in which $R'_1$ represents an alkyl radical having from 1 to 5 carbon atoms.

4. Process according to claim 1, characterised in that 5-R,S-trans amino 3,4,5,6-tetrahydro 1H-cyclohept [c,d] indol-6-ol, is prepared, as well as its salts of addition with acids.

5. Process according to claim 1, characterised in that 5-R,S-trans 5-methylamino 3,4,5,6-tetrahydro 1H-cyclohept [c,d] indol-6-ol is prepared as well as its salts of addition with acids.

**Ansprüche** (für die Vertragsstaaten : BE, CH, DE, FR, GB, IT, LI, LU, NL, SE)

1. Cycloheptindolol-Derivate der allgemeinen Formel I

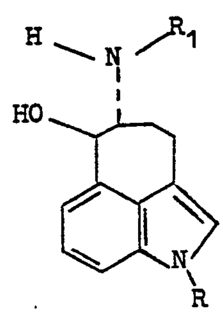

(I)

worin R ein Wasserstoffatom, einen Alkylrest mit 1 bis 5 Kohlenstoffatomen oder einen Aralkylrest mit 7 bis 12 Kohlenstoffatomen, gegebenenfalls substituiert durch Chlor-, Brom-, Methyl-, Äthyl-, Methoxy-, Trifluormethyl- oder Methylthioreste bedeutet und $R_1$ ein Wasserstoffatom, einen Alkylrest mit 1 bis 5 Kohlenstoffatomen, Cycloalkylalkyl mit 4 bis 7 Kohlenstoffatomen oder Aralkyl mit 7 bis 12 Kohlenstoffatomen, gegebenenfalls substituiert durch Chlor-, Brom-, Methyl-, Äthyl-, Methoxy-, Trifluormethyl- oder Methylthioreste, bedeutet sowie die Additionssalze mit Mineral- oder organischen Säuren dieser derivate.

2. Cycloheptindolol-Derivate der Formel I gemäß Anspruch 1, worin R und $R_1$ ein Wasserstoffatom oder einen Alkylrest mit 1 bis 5 Kohlenstoffatomen bedeuten, sowie die Additionssalze mit Mineral- oder organischen Säuren dieser Derivate.

3. 5R,S-trans-Amino-3,4,5,6-tetrahydro-1H-cyclohept-[c,d]-indol-6-ol sowie dessen Additionssalze mit Mineral- oder organischen Säuren.

4. 5R,S-trans-5-methylamino-3,4,5,6-tetrahydro-1H-cyclohept-[c,d]-indol-6-ol sowie dessen Additionssalze mit Mineral- oder organischen Säuren.

5. Verfahren zur Herstellung der Derivate der Formel I gemäß Anspruch 1 sowie von deren Additionssalzen mit Mineral- oder organischen Säuren, dadurch gekennzeichnet, daß man ein Produkt der Formel II

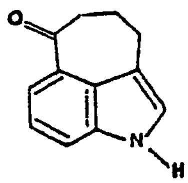

(II)

der Einwirkung eines Halogenids der Formel III

Hal—R' (III)

worin Hal ein Chlor-, Brom- oder Jodatom bedeutet und R' die Bedeutung von R mit Ausnahme von Wasserstoff hat, unterzieht, um ein Produkt der Formel IV

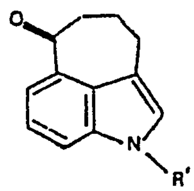

(IV)

zu erhalten, danach das Produkt der Formel II oder IV der Einwirkung von tert.-Butylnitrit in Gegenwart von Chlor wasserstoffsäure unterzieht, um ein Produkt der Formel V

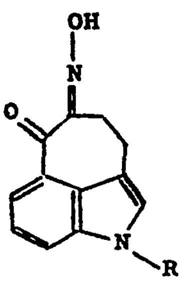

(V)

zu erhalten, welches man reduziert, um ein produkt der Formel $I_A$

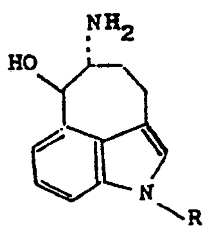

($I_A$)

worin R die angegebene Bedeutung besitzt, zu erhalten, das einem Produkt der Formel I entspricht, worin R wie vorstehend definiert ist und $R_1$ ein Wasserstoffatom bedeutet, danach

— entweder es isoliert, und gewünschtenfalls das Produkt der Formel $I_A$ in ein Salz überführt,

— oder das Produkt der Formel $I_A$ mit Trifluoressigsäureanhydrid, vorzugsweise in Gegenwart einer Base behandelt, um ein Produkt der Formel VI

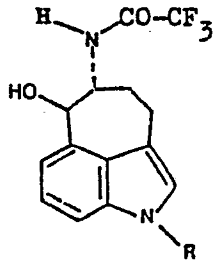

(VI)

worin R die angegebene Bedeutung besitzt, zu erhalten, das man mit einem Halogenid der Formel VII

$$Hal—R'_1 \qquad (VII)$$

umsetzt, worin Hal ein Chlor-, Brom- oder vorzugsweise Jodatom bedeutet und $R'_1$ die angegebene Bedeutung von $R_1$ mit Ausnahme von wasserstoff besitzt, um ein Produkt der Formel VIII

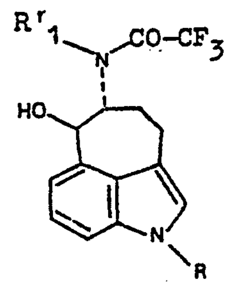

(VIII)

zu erhalten, worin R und $R'_1$ die angegebene Bedeutung besitzen, welches man einem Mittel unterzieht, das befähigt ist, das Trifluoressigsäureamid zu hydrolysieren, um ein Produkt der Formel $I_B$

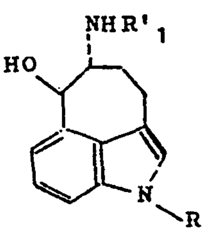

($I_B$)

zu erhalten, worin R und $R'_1$ die angegebene Bedeutung besitzen, das einem Produkt der Formel I entspricht, worin $R_1$ die Bedeutung von $R'_1$ besitzt, welches Produkt der Formel $I_B$ man isoliert und gewünschtenfalls in ein Salz überführt,

— oder das Produkt der Formel $I_A$ mit Alkylhaloformiat behandelt, um ein Produkt der Formel IX

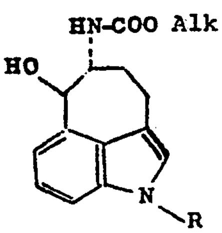

(IX)

worin R wie vorstehend definiert ist und Alk einen Alkylrest mit 1 bis 4 Kohlenstoffatomen bedeutet, zu erhalten, welches man reduziert, um ein Produkt der Formel $I_C$

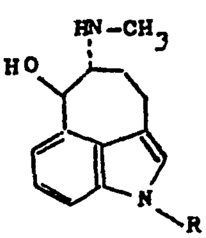

($I_C$)

worin R wie vorstehend definiert ist entsprechend einem Produkt der Formel I, worin $R_1$ einen Methylrest bedeutet, zu erhalten, welches Produkt der Formel $I_C$ man isoliert und gewünschtenfalls in ein Salz überführt,

— oder das Produkt der Formel $I_A$ mit einem Acetylierungsmittel behandelt, um so ein Produkt der Formel X

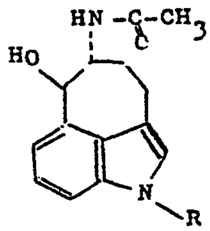

(X)

zu erhalten, worin R wie vorstehend definiert ist, welches man reduziert, um ein Produkt der Formel $I_D$

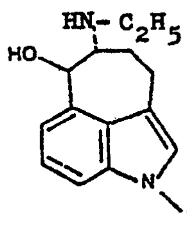

($I_D$)

zu erhalten, worin R wie vorstehend definiert ist, das einem Produkt der Formel I entspricht, worin $R_1$ einen Äthylrest bedeutet, welches Produkt der Formel $I_D$ man isoliert und gewünschtenfalls in ein Salz überführt,

— oder das Produkt der Formel $I_A$ mit Aceton in reduzierendem Milieu behandelt, um ein Produkt der Formel $I_E$

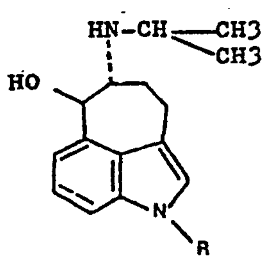

($I_E$)

zu erhalten, worin R wie vorstehend definiert ist, das einem Produkt der Formel I entspricht, worin $R_1$ einen Isopropylrest bedeutet, welches Produkt der Formel $I_E$ man isoliert und gewünschtenfalls in das Salz überführt.

6. Arzneimittel, dadurch gekennzeichnet, daß sie aus den Cycloheptindolol-Derivaten der Formel I gemäß Anspruch 1 oder aus deren Additionssalzen mit pharmazeutisch verträglichen Mineral- oder organischen Säuren bestehen.

7. Arzneimittel, dadurch gekennzeichnet, daß sie aus den Cycloheptindolol-Derivaten gemäß Anspruch 2 oder deren Additionssalzen mit pharmazeutisch verträglichen Mineral- oder organischen Säuren bestehen.

8. Arzneimittel, dadurch gekennzeichnet, daß sie aus den Cycloheptindolol-Derivaten gemäß Anspruch 3 oder 4 oder aus deren Additionssalzen mit pharmazeutisch verträglichen Säuren bestehen.

9. Pharmazeutische Zusammensetzungen, dadurch gekennzeichnet, daß sie als Wirkstoff zumindest eines der Arzneimittel gemäß einem der Ansprüche 6, 7 oder 8 enthalten.

**Ansprüche** (für den Vertragsstaat AT)

1. Verfahren zur Herstellung von Cycloheptindolol-Derivaten der allgemeinen Formel I

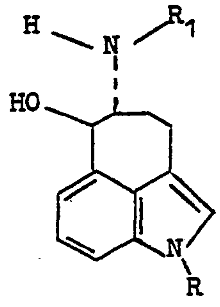

(I)

worin R ein Wasserstoffatom, einen Alkylrest mit 1 bis 5 Kohlenstoffatomen oder einen Aralkylrest mit 7 bis 12 Kohlenstoffatomen, gegebenenfalls substituiert mit Chlor-, Brom-, Methyl-, Äthyl-, Methoxy-, Trifluormethyl- oder Methylthioresten, bedeutet und $R_1$ ein Wasserstoffatom, einen Alkylrest mit 1 bis 5 Kohlenstoffatomen, einen Cycloalkylalkylrest mit 4 bis 7 Kohlenstoffatomen oder einen Aralkylrest mit 7 bis 12 Kohlenstoffatomen, gegebenenfalls substituiert durch Chlor-, Brom-, Methyl-, Äthyl-, Methoxy-, Trifluormethyl- oder Methylthioreste, bedeutet, sowie von deren Additionssalzen mit Mineral- oder organischen Säuren, dadurch gekennzeichnet, daß man ein Produkt der Formel II

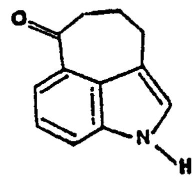

(II)

der Einwirkung eines Halogenids der Formel III

Hal—R' (III)

worin Hal ein Chlor-, Brom- oder Jodatom bedeutet und R' die Bedeutung von R mit Ausnahme von Wasserstoff besitzt, unterzieht, um ein Produkt der Formel IV

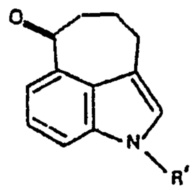

(IV)

zu erhalten, dann das Produkt der Formel II oder IV der Einwirkung von tert.-Butylnitrit in Gegenwart von Chlor wasserstoffsäure unterzieht, um ein Produkt der Formel V

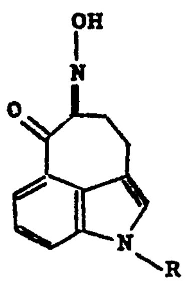

(V)

zu erhalten, welches man reduziert, um ein Produkt der Formel $I_A$

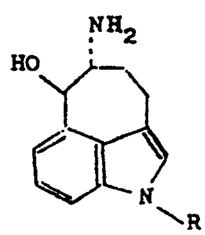

($I_A$)

worin R die angegebene Bedeutung besitzt, zu erhalten, das einem Produkt der Formel I entspricht, worin R wie vorstehend definiert ist und $R_1$ ein wasserstoffatom bedeutet, danach

— dieses entweder isoliert und gewünschtenfalls das Produkt der Formel $I_A$ in ein Salz überführt,

— oder dieses Produkt der Formel $I_A$ mit Trifluoressigsäureanhydrid, vorzugsweise in Gegenwart einer Base behandelt, um ein Produkt der Formel VI

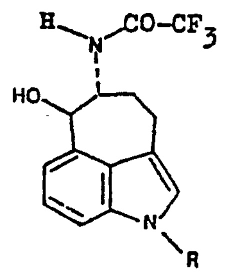

(VI)

zu erhalten, worin R die angegebene Bedeutung besitzt, welches man mit einem Halogenid der Formel VII

$$Hal—R'_1 \qquad (VII)$$

worin Hal ein Chlor-, Brom- oder vorzugsweise Jodatom bedeutet und $R'_1$ die angegebene Bedeutung von $R_1$ mit Ausnahme von Wasserstoff besitzt, umsetzt, um ein Produkt der Formel VIII

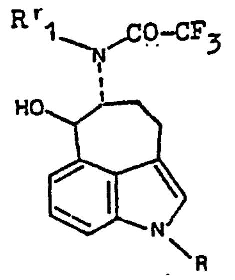

(VIII)

zu erhalten, worin R und $R'_1$ die angegebene Bedeutung besitzen, welches man einem Mittel unterzieht, das befähigt ist, das Trifluoressigsäureamid zu hydrolysieren, um ein Produkt der Formel $I_B$

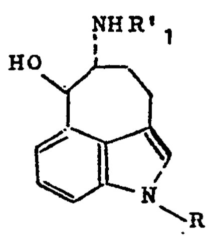

($I_B$)

zu erhalten, worin R und $R'_1$ die angegebene Bedeutung besitzen, das einem Produkt der Formel I entspricht, worin $R_1$ die Bedeutung von $R'_1$ besitzt, welches Produkt der Formel $I_B$ man isoliert und gewünschtenfalls in ein Salz überführt,

— oder das Produkt der Formel $I_A$ mit einem Alkylhaloformiat behandelt, um ein Produkt der Formel IX

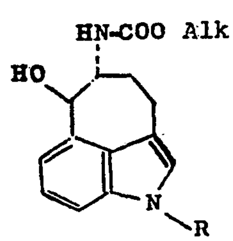

(IX)

worin R wie vorstehend definiert ist und Alk einen Alkylrest mit 1 bis 4 Kohlenstoffatomen bedeutet, zu erhalten, welches man reduziert, um ein Produkt der Formel $I_C$

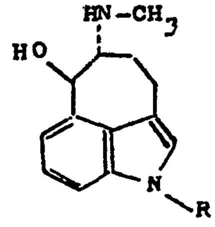

($I_C$)

worin R wie vorstehend definiert ist, zu erhalten, das einem Produkt der Formel I entspricht, worin $R_1$ einen Methylrest bedeutet, welches Produkt der Formel $I_C$ man isoliert und gewünschtenfalls in ein Salz überführt,

— oder das Produkt der Formel $I_A$ mit einem Acetylierungsmittel behandelt, um ein Produkt der Formel X

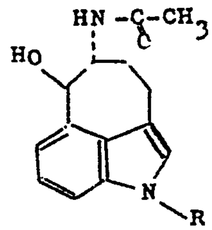

(X)

zu erhalten, worin R wie vorstehend definiert ist, welches man reduziert, um ein Produkt der Formel $I_D$

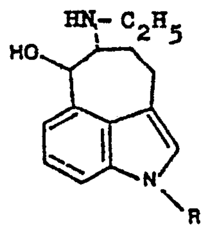

($I_D$)

worin R wie vorstehend definiert ist, zu erhalten, das einem Produkt der Formel I entspricht, worin $R_1$ einen Äthylrest bedeutet, welches Produkt der Formel $I_D$ man isoliert und gewünschtenfalls in ein Salz überführt,

— oder das Produkt der Formel $I_A$ mit Aceton in reduzierendem Milieu behandelt, um ein Produkt der Formel $I_E$

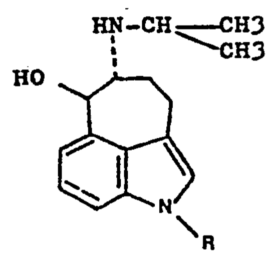

($I_E$)

worin R wie vorstehend definiert ist, zu erhalten, das einem Produkt der Formel I entspricht, worin $R_1$ einen Isopropylrest bedeutet, welches Produkt der Formel $I_E$ man isoliert und gewünschtenfalls in ein Salz überführt.

2. Verfahren gemäß Anspruch 1, dadurch gekennzeichnet, daß man ausgeht von einem Halogenid der Formel III, worin R' einen Alkylrest mit 1 bis 5 Kohlenstoffatomen bedeutet.

3. Verfahren gemäß Anspruch 1 oder 2, dadurch gekennzeichnet, daß man von einem Halogenid der Formel VII ausgeht, worin $R'_1$ einen Alkylrest mit 1 bis 5 Kohlenstoffatomen bedeutet.

4. Verfahren gemäß Anspruch 1, dadurch gekennzeichnet, daß man 5R-S-trans-Amino-3,4,5,6-tetrahydro-1H-cyclohept-[c,d]-indol-6-ol sowie dessen Addtionssalze mit Säuren herstellt.

5. Verfahren gemäß Anspruch 1, dadurch gekennzeichnet, daß man 5R,S-trans-5-Methylamino-3,4,5,6-tetrahydro-1H-cyclohept-[c,d]-indol-6-ol sowie dessen Additionssalze mit Säuren herstellt.